# EUROPEAN PATENT SPECIFICATION

(11) **EP 3 331 514 B1**
(45) Date of publication and mention of the grant of the patent: **16.10.2019**
(21) Application number: 16750740.9
(22) Date of filing: 04.08.2016
(51) Int. Cl.: A61K 31/167, A61K 31/196, A61P 29/00, A61K 9/00, A61P 17/02, A61K 47/14, A61K 9/06

(54) **RECTAL TOPICAL LIPOGEL COMPRISING AN ANESTHETIC AGENT AND A NON-STEROIDAL ANTI-INFLAMMATORY AGENT FOR PAIN RELIEF**
REKTALES TOPISCHES LIPOGEL MIT EINEM ANÄSTHESIEMITTEL UND EINEM NICHTSTEROIDALEN ENTZÜNDUNGSHEMMER ZUR SCHMERZLINDERUNG
LIPOGEL RECTALE TOPIQUE COMPRENANT UN AGENT ANESTHÉSIQUE ET UN AGENT ANTI-INFLAMMATOIRE NON STÉROÏDIEN POUR LE SOULAGEMENT DE LA DOULEUR

(30) Priority: 05.08.2015 EP 15382416
(43) Date of publication of application: 13.06.2018
(73) Proprietor: Fundació Institut d'Investigació Biomèdica de Bellvitge (IDIBELL), 08908 L'Hospitalet de Llobregat (ES)
(72) Inventor: LINARES GIL, Mª José, 08908 Hospitalet del Llobregat (ES)
(74) Representative: ZBM Patents - Zea, Barlocci & Markvardsen
(86) International application number: PCT/EP2016/068618
(87) International publication number: WO 2017/021481

(56) References cited:
- US-A1- 2005 256 187
- MOJGAN RAHIMI ET AL: "Comparison of topical anesthetic cream (EMLA) and diclofenac suppository for pain relief after hemorrhoidectomy: a randomized clinical trial", SURGERY TODAY ; OFFICIAL JOURNAL OF THE JAPAN SURGICAL SOCIETY, SPRINGER-VERLAG, TO, vol. 42, no. 12, 19 June 2012 (2012-06-19) , pages 1201-1205, XP035137502, ISSN: 1436-2813, DOI: 10.1007/S00595-012-0222-9
- Roja Rahimi ET AL: "A Systematic Review of the Topical Drugs for Post Hemorrhoidectomy Pain", International Journal of Pharmacology, 1 October 2012 (2012-10-01), pages 628-637, XP055307519, DOI: 10.3923/ijp.2012.628.637 Retrieved from the Internet: URL:http://www.scialert.net/qredirect.php? doi=ijp.2012.628.637&linkid=pdf [retrieved on 2016-10-04]
- IRER B ET AL: "Diclofenac suppository administration in conjunction with lidocaine gel during transrectal ultrasound-guided prostate biopsy: Prospective, randomized, placebo-controlled study", UROLOGY, BELLE MEAD, NJ, US, vol. 66, no. 4, 1 October 2005 (2005-10-01), pages 799-802, XP027690481, ISSN: 0090-4295 [retrieved on 2005-10-01]
- Anonymous: "PACKAGE LEAFLET: INFORMATION FOR THE USER EMLA Cream 5%", , 1 June 2012 (2012-06-01), XP055307970, Retrieved from the Internet: URL:https://www.medicines.org.uk/emc/PIL.3 478.latest.pdf [retrieved on 2016-10-05]

## Description

The invention relates to a topical gel composition comprising a local anesthetic agent and a non-steroidal anti-inflammatory agent, for use in the treatment of pre- and postoperative pain in the benign anorectal surgery, particularly, by topical rectal administration and using a specific dosage regime. It also relates to a specific topical lipogel composition which is especially suitable for this use.

### BACKGROUND ART

Anorectal surgery is often a painful cure. Effective pain control is essential for recovery, improved wound healing, and reduced hospital admission rates. Anesthetic agents that are able to inhibit the excitatory process in the nerve ending are often used to reduce or block the perception of pain. The two key elements of an anesthetic agent in pain control are the anesthetic potency and the duration of action. These elements are related to the lipid and protein structure of the nerve membrane. Anesthetic potency is correlated with lipid solubility. Anesthetic duration is related to the degree of protein binding at the membrane level, the greater the binding activity, the longer duration of anesthesia. Apart from the lipid and protein structure of the nerve membrane, there are other factors that also play a role in the anesthetic effects of various agents such as the pH and the type and size of the nerve fibers. Local anesthetics are more effective in more basic pH environments. Smaller nerves with lighter myelin coatings are more easily blocked than the larger and more heavily myelinated ones.

Local anesthetics are routinely used and are extremely effective for completely blocking pain sensation during surgery. Administered in the form of a pudendal block, coupled with direct injections into the operative sites before beginning the operation, local anesthetics are also effective in managing pain in the postoperative setting. Their benefit, however, is limited by the duration of effectiveness. Bupivacaine and lidocaine are the most common local anesthetic agents used in anorectal procedures. Bupivacaine and lidocaine have established durations of action of less than 12 hours, even with the use of epinephrine. Because of this, there have been several attempts to improve the duration profile of local anesthetics.

Elastomeric pain pumps and catheter systems have shown to help to ameliorate the pain for several days postoperatively. Ideally, the pumps should deliver a steady dose of the anesthetic for one to seven days, depending on the size of the pump. However, these systems may cause several technical problems such as catheter dislodgement and infections. Epidural anesthesia is a reliable way to ensure a pain-free postoperative recovery. The catheters can remain in place for up to five days after a procedure. Although they are most often used in the hospital setting, it is theoretically possible to use this type of system on an outpatient basis. Adoption of this technique has been hampered by the need for periodic infusion of the anesthetic, catheter dislodgement, various systemic side effects, and the potential for a delay in the diagnosis of a possibly serious catheter-related infection. Finally the topical application of local anesthetics is of marginal and unpredictable efficacy.

Currently, there are numerous specialties containing lidocaine as a local anesthetic and many other anti-inflammatory containing diclofenac. As to lidocaine, it can be in semi-solid form (cream or ointment) associated to fluocinolone for rectal application for the treatment of internal and external hemorrhoids in addition to conventional topical forms. There is also a rectally administrable product containing only lidocaine as anesthetic which is used to treat internal hemorrhoids, anal fistulas, proctitis, and perianal eczema. This preparation is presented as an ointment. In addition, according to the abstract of the Scientific Information database (SID), MR. Rafiei et al., disclose in "Comparison of Post Operative Analgesia with preoperative topical lidocaine gel and lubricant gel in hemorroidectomy operation", Iranian Journal of Surgery 2008, vol. 15, pp.28-33, the use of a preoperative lidocaine gel in hemorrhoidectomy operation to reduce the pain intensity and prolong the periods without pain.

Moreover, currently it is used, among other anti-inflammatory agents, diclofenac, which has analgesic, antipyretic and anti-inflammatory activity being the anti-inflammatory effect greater than its analgesic effect. It can be applied rectally as suppositories, creams and ointments. Current creams and ointments on the market containing diclofenac have not been validated for the analgesic treatment of a surgical wound i.e. they are not considered suitable for the administration in case of wounds because of the fact that they contain irritating excipients that may cause mucosal injury. On the other hand, the current creams and ointments on the market containing corticoids for use in anorectal conditions are contraindicated for the treatment of wounds because they delay the healing and favor the over infection. As suppositories, creams also have the disadvantage that occupies a lot of space in the rectum and produce burning and increased contractility of the anal sphincter. Besides, the great majority of technical sheets of diclofenac, either contraindicate or advise against the use of diclofenac in situation of proctitis, which occurs when surgery or when there are acute symptoms of internal hemorrhoids, anal fistulas, and fissures.

C. Placer et al discloses the results of a clinical study which shows that a unique dose of 100 mg of topical rectal diclofenac in the form of a suppository, administered to the anesthetized patient by a surgeon at the end of the hemorrhoid surgery, reduces the need for rescue analgesia and the frequency of presenting urinary retention as a post-surgery complication (cf. C. Placer et al., "A single dose of intrarectal diclofenac reduces urinary retention after surgery for hemorrhoids. Results of a randomized controlled trial" in Spanish, Cir. Esp. 2008 vol. 83, pp. 301-5). Unfortunately, since suppositories may cause irritation and pain, they are not suitable for the administration in case of wounds. For this reason in this study an only single dose was administered by the surgeon taking benefit that the patient was anesthetized.

Mojgan Rahimi et al., in "Comparison of topical anesthetic cream (EMLA) and diclofenac suppository for pain relief after hemorrhoidectomy: a randomized clinical trial", Surg Today 2012, vol. 42; pp.1201-1205 compares the efficacy and side effects of diclofenac and topical eutectic mixture of local anesthetics (EMLA) for pain relief after hemorrhoidectomy. It concludes that the use of topical EMLA cream is appropriate for short-term pain control following surgery and that the analgesic effects fo the diclofenac suppository were more sustainable for the later time points compared to EMLA. Although the suppository is used once the surgery is complete, it is only used once the day of the surgery since it can be administered under the effects of the anesthetic. Suppositories are not suitable for the administration in case of wounds because of the irritation and pain.

B. Irer et al., in "Diclofenac suppository administration in conjunction with lidocaine gel during transrectal ultrasound-guided prostate biopsy: prospective, randomized, placebo-controlled study" Adult Urology 2005, vol. 66, pp.799-802 describes diclofenac suppository administration in conjuntion with lidocaine gel during transrectal ultrasound-guided prostate biopsy to reduce pain and improve patients tolerance. However, the pain of a biopsy is not comparable to the postoperative pain of a surgical wound.

On the other hand, the combination of blocking sodium channels of the class of local anesthetics and an AINE has been described for the treatment of neuromuscular pain. Thus, US2005/0256187 discloses a pharmaceutical composition comprising blocking sodium channels of the class of local anesthetics of ester or amide type and an AINE for the treatment of neuromuscular pain. Also, Liedtke et al., in "Pharmacological Concept for Topical Synergistic Analgesia of Peripheral Neuromuscular Pain", Drug Res 2006, vol. 56, pp.108-114 discloses a combination of lidocaine and diclofenac which shows a topical synergistic analgesia of peripheral neuromuscular pain. Therefore, these documents disclose the use of the combination for the treatment of somatic type pain.

However, the anorectal pain has significant differentiating features with respect to neuromuscular or musculoskeletal pain since it is a kind of a pain with visceral and autonomous sensitivity, resulting in visceral pain type with involvement of the autonomic nervous system, which is absent in somatic or neuromuscular pain.

There are also anatomic-physiological and molecular differences between somatic and visceral pain. An essential feature of visceral pain, unlike somatic pain, is that one sensory neuron at the dorsal horn of the spinal cord is implicated in the sensitivity of various visceras, yielding to a diffuse pain and badly localized which is reflected in different areas from where the injury occurs. In contrast, neuromuscular pain is well located. Another characteristic of the benign anorectal post-operative pain is that from the third day may be caused by the presence of secondary infection in the operated area which is accepted *per* se as contaminated.

The number of neurons and somatic sensitve fibers located in the skin that modulates the somatic sensitivity is of about one million, while the ones that modulate visceral sensitivity of the viscera surface are only a 5%.

The spinothalamic tract and dorsal column represent the main ascending somatosensory pathway. The spinothalamic tract plays a key role in somatic pain and its lesion solves the non- treatable somatic pain, but it does not affect to visceral pain which is conducted by the dorsal column and its lesion reduces significatively the visceral pain in humans.

An important difference between the cutaneous somatic afferent pathway and the visceral one is the size and distribution of the diferent nervous fibers and also their thickness.The visceral afferent fibers thickness is ¼ part of the thickness of the somatic afferent pathway. Despite this, there is no an increase in the number of the transmited stimulus by the polymodal mucous receptors, muscle or serose, which are very sensitive to the chemical stimulus and to the intraluminar pressure changes. The visceral sensitivity is mainly mechanical because of the distension (with a pressure greater than 45 mm Hg is painful) and chemical which is produced by the isquemia due to the vascular contraction and/or other chemical stimulus. On the contrary, the cutaneous and somatic sensitivity is due to the heat and cold and to the chemical changes.

Moreover, there are several differences in the receptors between visceral pain and somatic pain. See table below:

| | **Visceral sensitivity** | **Somatic sensitivty** |
|---|---|---|
| Afferent ventral roots | Quite common | Not common |
| Amielinic C fibers | Predominance | Only 60-70% (but for touch) |
| Aβ / *A*δ thick fibers | Very scarce / scarce | Very abundant /abundant |
| Receptors | Mecanical receptors Chemoreceptors | Thermoreceptors Chemoreceptors |
| Silent receptors | Abundant (90%)* | (20%)* |
| Vasoactive intestinal polypeptide (VIP)** | abundant | rare |
| Nitrous oxide (NO) | abundant | Little common |
| trkA and B receptors | Abundant (90%) | Litte common and uniform distribution trk A, trk Band trk C |
| trk C receptors | Rare (10%) | |

| | | |
|---|---|---|
| * cat ** Very frequent in intestines and rectum | | |

Thus, the somatic and the visceral pain show different features and, therefore, it cannot be predicted that a treatment that is effective for somatic or skeletal muscular pain will also be effective for the treatment of benign anorectal pain, a kind of pain that has a visceral and autonomous sensitivity. For instance, diltiazem is effective and reduces pain and posthemorrodeictomía derivative chronic fissure, and therefore, reduces pre and post- surgical pain. However, it provokes neuromuscular pain as a side effect, (cf. http://www.mayoclinic.org/drugs-supplements/diltiazem-oral-route/side-effects/drg-20071775). It can also provoke body aches or pain such as back pain, arthralgia, headache from 3 % up to 10% (cf. J. T: Wright el al., "Antihypertensive efficacy of night-time graded-release diltiazem versus morning amlodipine in Africans Americans" AJH 2004, vol. 17, pp. 734-742, in particular table 4), and moderate to severe myalgia when patients take both diltiazem and simvastatin (cf. S. Guis et al., Drug-induced and toxic myopathies. Best practices & Research Clinical Rheumathology 2003, vol. 17, pp. 877-907). Likewise, capsaicin is effective in somatic and neuropathic pain, but concerning anorectal pain, not only does not produce any improvement but even can produce visceral hyperalgesia in patients (cf. P. Arnand et al., "Topical capsaicin for pain management: therapeutic potential and mechanisms of action of the new high-concentration capsaicin 8% patch" British Journal of Anaesthesia, 2011, vol. 107, pp. 490-502) Finally, corticoids widely used in form of ointments and creams for the pre-operative anorectal pain, are contraindicated when there is a wound, since they cause delay in healing and favor the over infection (cf. J. Listing et al., "The risk of infections associated with rheumatoid arthritis, with its comorbidity and treatment", Rheumatology, 2013; vol. 52, pp. 53-61; W. G. Dixon et al., "The influence of systemic glucocorticoid therapy upon the risk of non-serious infection in older patients with rheumatoid arthritis: a nested case-control study" Ann Rheum Dis 2011; vol. 70, pp. 956-960. 41; or M.S. Lionakis et al., "Glucocorticoids and invasive fungal infections", Lancet 2003; vol. 362: pp. 1828-38).

In short, postoperative pain in general and specifically, the one that occurs after benign anorectal surgery remains today as a complex and difficult problem, which must be addressed taking into account their distinctive anatomical, pathophysiological, and molecular implications. Therefore, there is still a need to find an appropriate treatment to relief the pain in patients that are submitted to a benign anorectal surgery.

### SUMMARY OF THE INVENTION

Inventors have found that pre- and postoperative anorectal pain due to benign anorectal surgery can be treated by anorectal administration twice per day during three days and once per day from the fourth day of treatment with a topical gel composition comprising lidocaine or its pharmaceutically acceptable salts (topical anesthetic) and diclofenac or its pharmaceutically acceptable salts (non-steroidal anti-inflammatory drug). It is advantageous because the composition of the present invention can be administered directly at the location of a pain-inducing lesion and can be used as a main analgesic in the treatment of pre- and postoperative anorectal pain due to benign anorectal surgery. Thus, it allows using diclofenac in a surgical wound without the patient being anesthetised, for a period of a week using a specific dosage regime.

As it is illustrated in the Examples in a comparative way, both a topical gel composition comprising lidocaine hydrochloride and diclofenac sodium according to the present invention and a topical gel composition comprising only lidocaine hydrochloride are effective in the treatment of pre- and postoperative anorectal pain due to benign anorectal surgery. However, by the use of a topical gel composition comprising lidocaine and diclofenac according to the present invention, the patients experimented a mean reduction of the pain intensity, evaluated with visual analogyc scale (VAS), nearly double than the one experimented by the use of a topical gel composition comprising only lidocaine. The results were statistically significant.

Therefore, with the treatment of the present invention the level of pain is lower, resulting in average pain intensity measured with the VAS lower than with the conventional analgesic pattern and the threshold of pain increases, facilitating its control. There was no infection complication on the surgical wound, nor pain by infection, that can be responsible of the late pain in this type of surgery. Therefore, this treatment allows reducing the oral and parenteral analgesics consumption. Advantageously, the topical gel composition may be administered by the patient both during his hospitalization and after he is delivered from hospital, administered by him at home, which is a more satisfactory treatment for the patient. Moreover, this also implies a reduction in medical costs due to the hospital assistance. It also reduces the expense because it avoids prolonging the hospitalary stay to control the pain. Besides, the administration by the own patient allows an individualized and prolonged treatment, and makes the treatment more flexible in function of the needs of the patient.

Inventors have also found that a topical composition comprising lidocaine and diclofenac, or any of the pharmaceutically acceptable salts of each of them, as well as a medium chain triglyceride, colloidal silica, soy lecithin, and Marigold extract, is specially suitable for the treatment of pre- and postoperative anorectal pain due to benign anorectal surgery because it is highly effective and has great tolerance. It is also homogeneous and stable.

On the one hand, the composition is highly effective because the two substances that act in alleviating pain have different points of attack as regards tissue topology which means a benefit of an additive effect of an anesthetic and anti-inflammatory action. Besides, the components of the composition make it to have analgesic, anti-inflammatory, antiseptic, antimicrobial, antimicotic and antiviric activity, as well as a physiological healing and regenerative ability of the integrity of the mucous membrane and skin. Therefore, it opens a new way of treating pain in an integral way treating the cause that intensify the pain, such as inflammation, infection, healing disorders and delays, and preventing the pain due to an over infection of the healing, which appears at the fourth day of treatment.

On the other hand, the composition shows a great tolerance because it is in the form of lipogel. A lipogel is generally better tolerated at a mucous level and it is more physiological due to the rectal membranes are lipophiles. The lipogel of the invention is self-emulsionable in the rectal mucous which results in an emulgent and lubricant action over the anorectal tract in which it acts. The good physico-chemical and organoleptical properties of the lipogel have been achieved using only natural excipients.

Advantageously, the lipofilc gel has a viscosity lower than the rectal mucous and has an adhesive capacity, without increasing the pressure over the rectal walls. Besides, it has a lubricant effect. The lipogel of the invention is able to cross the rectal mucous and be adhered to the mucous for its topical action forming a film over the anorectal mucous having a long term action. The improved tolerance is also due to the fact that the composition may be free of irritant excipients, that is, components generally used in the compositions of the prior art but that are known to have a negative effect for the treatment of wounds. Thus, for instance, the topical gel of the invention is free of thickeners such as polyethylene glycol (PEG) and/or its derivatives (esters of polyethylene glycol derivatives such as polyoxyethylene polyoxyethylene stearates, or polyethylene glycol succinate); of ethylendiamino tetraacetic acid (EDTA) and/or its derivatives such as ethylendiamine tetraacetic disodium acid; EDTA-4NA; of preservatives such as parabens; phenoxyethanol or triclosan; and/or free of phthalates (DEP); silicones; glycerine; or lanolin, as well as of corticoids.

Finally, the fact that the gel is a lipogel avoids the incompatibility in water of the lidocaine hydrochloride and the sodium diclofenac due to the decomposition of salts.

Thus, a first aspect of the present invention relates to a pharmaceutical composition in the form of a lipogel comprising a therapeutically effective amount of lidocaine or a pharmaceutically acceptable salt thereof, and a therapeutically effective amount of diclofenac or a pharmaceutically acceptable salt thereof, together with one or more pharmaceutically acceptable excipients or carriers, for use in the treatment of pre- and post-operative pain in benign anorectal surgery, wherein the treatment comprises the topical rectal administration of the composition twice per day during three days and once per day from the fourth day of treatment. Generally, the administration once per day is prolonged up to one or two weeks depending on the needs of the subject. The benign anorectal surgery is selected from hemorrhoids, anal fistulas, anal fissure, proctitis, and perianal eczema.

A second aspect of the present invention relates to a pharmaceutical composition which is in the form of a topical lipogel and which comprises: a) a therapeutically effective amount of lidocaine or a pharmaceutically acceptable salt thereof; b) a therapeutically effective amount of diclofenac or a pharmaceutically acceptable salt thereof; c) a medium chain triglyceride; d) colloidal silica; e) soy lecithin; and f) Marigold extract. The topical lipogel may also comprise aloe vera and/or Asiatic centellum extract.

A third aspect of the present invention relates to a kit for use in the treatment of pre- and post-operative pain in benign anorectal surgery according to claim 15 comprising: a) a first pharmaceutical composition in the form of a lipogel comprising a therapeutically effective amount of lidocaine or a pharmaceutically acceptable salt thereof, and a therapeutically effective amount of diclofenac or a pharmaceutically acceptable salt thereof, together with one or more pharmaceutically acceptable excipients or carriers; and b) a second pharmaceutical composition comprising lidocaine or a pharmaceutically acceptable salt thereof, together with one or more pharmaceutically acceptable excipients or carriers.

### DETAILED DESCRIPTION

As mentioned above, in one aspect of the invention, it is provided a pharmaceutical composition in the form of a lipogel with the features mentioned above for use in the treatment of pre- and post-operative pain in benign anorectal surgery using a specific route of administration and a specific dosage regime.

Lidocaine is a common local anesthetic and class-1b antiarrhythmic drug. Lidocaine is used in oral, parenteral and topical formulations as local anesthetic. The efficacy profile of lidocaine as a local anesthetic is characterized by a rapid onset of action and intermediate duration of efficacy. It has the following chemical formula:

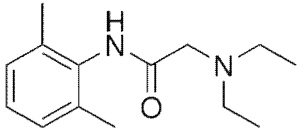

Diclofenac is a non-steroidal anti-inflammatory drug (NSAID) taken or applied to reduce inflammation and as an analgesic reducing pain in certain conditions. Diclofenac is also used in oral, parenteral and topical formulations. Its chemical name is 2-(2,6-dichloranilino) phenylacetic acid and has the following chemical formula:

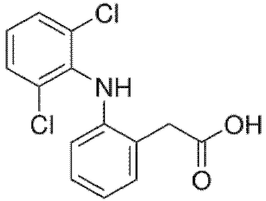

The term "pharmaceutically acceptable salts" used herein encompasses any salt formed from pharmaceutically acceptable non-toxic acids or bases including inorganic or organic acids. There is no limitation regarding the salts, except that if used for therapeutic purposes, they must be pharmaceutically acceptable.

As lidocaine is a basic compound, salts may be prepared from pharmaceutically acceptable non-toxic acids, including inorganic and organic acids. Such acids include, for instance, acetic, benzenesulfonic, benzoic, camphorsulfonic, citric, ethansulfonic, fumaric, gluconic, glutamic, hydrobromic, hydrochloric, lactic, maleic, malic, mandelic, methanesulfonic, phosphoric, succinic, sulfuric, tartaric, or p-toluensulfonic acid. In a preferred embodiment, the hydrochloride salt of lidocaine is used.

As diclofenac is an acid compound, salts may be prepared from pharmaceutically acceptable non-toxic bases, including inorganic and organic bases. Diclofenac can exist in a variety of salt forms, including sodium, potassium, or diethylamine forms. In a preferred embodiment, the sodium salt of diclofenac is used.

Both lidocaine and diclofenac, as well as, their pharmaceutically acceptable salts may be in crystalline form either as free solvation compounds or as solvates, e.g. hydrates, and it is intended that both forms are within the scope of the present invention. Methods of solvation are generally known within the art.

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

Lipogels are physico-chemical structures which, if obtained in clear form, enable formulations to be prepared having a consistency similar to or even better than creams or modern gel emulsions, without the use of compounds typical of aqueous systems.
The Lipogel of the invention has a particularly pleasing appearance, and compared with aqueous formulations have the advantage of not requiring preservatives and of being able to be used in very small quantity. The administration of lipogels is also more pleasant than traditional ointment formulations. It also shows good properties of extensivity and adherence to the skin. The lipogels of the present invention may generally have a viscosity equal to or less than 2000 miliPascals. Generally, the viscosity is between 1000-2000 miliPascals. The viscosity can be measured for instance with a Broncofield Viscosimeter such as Broncofield 2000 CAP, at 25°C and the conditions given in the examples.

In a preferred embodiment, the lipogel for use in the treatment of pre- and postoperative pain in benign anorectal surgery with the specific route of administration and the specific dosage regime disclosed above, comprises lidocaine in the form of hydrochloride salt and diclofenac in the form of sodium salt.

In another preferred embodiment, the lipogel of the invention is for use in a treatment which comprises the use of 300 milligrams of lidocaine hydrochloride and 75 milligrams of diclofenac sodium twice per day during three days, and 300 milligrams of lidocaine hydrochloride and 75 milligrams of diclofenac sodium once per day from the fourth day up to one or two weeks depending on the needs of the subject.

The term benign anorectal surgery includes internal hemorrhoids, anal fistulas, anal fissure, proctitis, and perianal eczema.

The topical pharmaceutical formulation may be used medically for humans or for animals in the veterinary area.

In another preferred embodiment, the lipogel of the invention is for use in a treatment as defined above which further comprises the topical administration of a second pharmaceutical composition comprising lidocaine or a pharmaceutically acceptable salt thereof, together with one or more pharmaceutically acceptable excipients or carriers, where the lipogel is administered once up to 30' before a bowel movement and once after the bowel movement, generally, just after the bowel movement although it depends on the needs of the patient.

With this combined treatment the level of pain is even lower and there is also a lower index of over infection of the surgical wound, which results in a more satisfactory treatment for the patient.

In a preferred embodiment, the lipogel for use in the treatment as defined above is free of irritant excipients, that is, components generally used in the compositions of the prior art but that are known to have a negative effect for the treatment of wounds. In a more preferred embodiment, the lipogel for use in the treatment as defined above is free of thickeners, for instance, polyethylene glycol or polyethylene glycol derivatives such as polyoxyethylene polyoxyethylene stearates, or polyethylene glycol succinate. In another more preferred embodiment, the lipogel for use in the treatment as defined above is free of ethylendiamino tetraacetic acid (EDTA) and/or its derivatives such as ethylendiamine tetraacetic disodium acid; EDTA-4NA. In another preferred embodiment, the lipogel for use in the treatment as defined above is free of preservatives, for instance, parabens; phenoxyethanol; or triclosan. In another more preferred embodiment, the lipogel for use in the treatment as defined above is free of phthalates (DEP), silicones, glycerine, or lanolin.

In another more preferred embodiment, the lipogel for use in the treatment as defined above is free of corticoids.

In another more preferred embodiment, the lipogel for use in the treatment as defined above is free of any of the previous compounds.

In another preferred embodiment, the lipogels for use in the treatments as defined above has a pH of 5.2 -7.2. In another more preferred embodiment, the lipogel of lidocain hydrochloride plus diclofenac sodium for use in the treatment as defined above has a pH of 6.8-7.2. In another more preferred embodiment, the lipogel of lidocaine hydrochloride plus diclofenac sodium for use in the treatment as defined above has a pH of 6.8-7.2 and the lipogel of lidocaine hydrochloride for use in the treatment as defined above has a pH of 5.2-6.2.

In another preferred embodiment, the composition for use in the treatment as defined above comprises a medium chain triglyceride; colloidal silica; soy lecithin; and marigold extract as excipients or carriers. In a more preferred embodiment, the composition further comprises aloe vera as excipient.

Medium chain triglycerides are triglycerides whose fatty acids have an aliphatic tail of 6-12 carbon atoms. The fatty acids found in medium chain triglycerides are called medium-chain fatty acids. Like all triglycerides, medium chain triglycerides are composed of a glycerol backbone and three fatty acids. In the case of medium chain triglycerides, 2 or 3 of the fatty acid chains attached to glycerol are medium-chain in length. Rich sources for commercial extraction of beneficial medium chain triglycerides include palm kernel oil and coconut oil.

Silica colloidal is a suspension of fine amorphous, non-porous, and typically spherical silica particles in a liquid phase. Generally, silica colloidal 200 is used in the compositions of the present invention. Preferably, anhydrous silica colloidal is used.

Soy lecithin 300 is a mixture of phosphate compounds, mainly consisting of phosphatidylcholine, phosphatidylserine and phosphatidylinositol.

Marigold oil comes from Callendula officinalis flower crushed and extracted with a vegetable oil. It has four main groups of constituents, namely flavonoids, polysaccharides, and volatile oil and triterpenes. The latter seems to represent the principal group, with many compounds isolated, including pentacyclic alcohols, glycosides (saponins) and sterols. In particular it contains flavonoids (Pharmacopoeial standard not less than 0.4% flavonoids; flavonol (isorhamnetin, quercetin) glycosides including isoquercitrin, narcissin, 3-neohesperidosides, and rutin); polysaccharides (three polysaccharides PS-I, -II and -III have a (1→3)-β-d-galactan backbone with short side chains at C-6, comprising α-araban-(1→3)-araban, α-I-rhamnan-(1→3)-araban or simple α-l-rhamnan moieties); terpenoids (many components, including α- and β-amyrin, lupeol, longispinogenin, oleanolic acid, arnidiol, brein, Marigold diol, erythrodiol, faradiol, faradiol-3-myristic acid ester, faradiol-3-palmitic acid ester,3 helantriols A1, B0, B1 and B2, lupeol, maniladiol, urs-12-en-3,16,21-triol, ursadiol; oleanolic acid saponins including calendulosides C-H;4 campesterol, cholesterol, sitosterol, stigmasterol and taraxasterol (sterols)); volatile oils (terpenoid components include menthone, isomenthone, caryophyllene and an epoxide and ketone derivative, pedunculatine, α- and β-ionone, a β-ionone epoxide derivative, dihydroactinidiolide); and other constituents (Calendulosoides,Faradiol-3-O-laurate, Faradiol-3-Omyristate, faradiol-3-Opalmitate, maniladiol-3-O-laurate, maniladiol-3-Omyristate, Bitter (Ioliolide), 7 arvoside A (sesquiterpene glycoside), 8 carotenoid pigments9 and calendulin (gum)).

Marigold flower consists of the whole or cut, dried, and fully opened flowers which have been detached from the receptacle of the cultivated, double-flowered varieties of Callendula officinalis. It contains not less than 0.4% of flavonoids, calculated as hyperoside (C₂₁H₂₀O₁₂ = 464.4) calculated with reference to the dried drug.

Aloe vera comes from the leaves of Aloe barbadensis. It contains at least 28% of hydoxyantracene derivatives. It is containing aloin chrysophanic acid, volatile oils and resins.

In a preferred embodiment, the lipogel for the use as defined above, comprises:
a) the lidocaine or the pharmaceutically acceptable salt thereof in an amount comprised between 1 and 3% by weight of the total weight of the composition; b) the diclofenac or the pharmaceutically acceptable salt thereof in an amount comprised between 0.5 and 2% by weight of the total weight of the composition; c) the medium chain triglyceride in an amount comprised between 70 and 90% by weight of the total weight of the composition; d) the colloidal silica in an amount comprised between 3 and 8 % by weight of the total weight of the composition; e) the soy lecithin in an amount comprised between 1 and 6% by weight of the total weight of the composition; f) the Marigold extract in an amount comprised between 0.5 and 2% by weight of the total weight of the composition; and g) the aloe vera in an amount comprised between 0.5 and 2% by weight of the total weight of the composition.

In a more preferred embodiment, the lipogel further comprises Centella asiatica extract. Centella asiatica extract comes from the fragmented and dried aerial parts of centella asiatica plant (Synonym: Hydrocotyle asiatica L.). It has as constituents: Amino acids (Alanine and serine (major components), aminobutyrate, aspartate, glutamate, histidine, lysine and threonine, the root contains greater quantities than the herb); flavonoids (Quercetin, kaempferol and various glycosides); terpenoids (Triterpenes, asiaticoside, centelloside, madecassoside, brahmoside and brahminoside, Aglycones are referred to as hydrocotylegenin A-E, compounds A-D are reported to be esters of the triterpene alcohol R1-barrigenol, Asiaticentoic acid, centellic acid, centoic acid and madecassic acid); volatile oils (various terpenoids including β-caryophyllene, trans-β-farnesene and germacrene D (sesquiterpenes) as major components, α-pinene and β-pinene); and other constituents (Hydrocotylin (an alkaloid), vallerine (a bitter principle), fatty acids (e.g. linoleic acid, linolenic acid, lignocene, oleic acid, palmitic acid, stearic acid), phytosterols (e.g. campesterol, sitosterol, stigmasterol), resin and tannin. The underground plant parts of hydrocotyle have been reported to contain small quantities of at least 14 different polyacetylenes.

Preferably, the centella asiatica extract is present in an amount comprised between 0.5 and 2% by weight of the total weight of the composition.

Additionally, the lipogel of the present invention may contain other ingredients, such as fragrances, colorants, and other components known in the state of the art for use in topical formulations.

Any commercial soy lecithin; Marigold extract; aloe vera, and centella asiatica extract can be used for the purposes of the invention.

In a particular embodiment, in combination with any of the embodiments disclosed above, the lipogel of the invention for use as defined above, comprises lidocaine or a pharmaceutically acceptable salt thereof in an amount comprised between 2% by weight of the total weight of the composition; and diclofenac or a pharmaceutically acceptable salt thereof in an amount comprised between 0.5% by weight of the total weight of the composition. Particularly, the active ingredients are lidocaine hydrochloride and diclofenac sodium.

In another particular embodiment, in combination with any of the embodiments disclosed above, the lipogel of the invention for use as defined above, comprises a medium chain triglyceride in an amount comprised between 80-90% by weight of the total weight of the composition. Preferably, the amount of the medium chain triglyceride is comprised between 85-90% by weight of the total weight of the composition.

In another particular embodiment, in combination with any of the embodiments disclosed above, the lipogel of the invention for use as defined above comprises colloidal silica in an amount comprised between 5% and 7 % by weight of the total weight of the composition; Preferably, the amount of silica colloidal is 6%% by weight of the total weight of the composition.

In another particular embodiment, in combination with any of the embodiments disclosed above, the lipogel of the invention for use as defined above comprises soy lecithin in an amount comprised between 1.5 and 3% by weight of the total weight of the composition.

In another particular embodiment, in combination with any of the embodiments disclosed above, the lipogel of the invention for use as defined above comprises Marigold extract in an amount comprised between 0.5 and 1.5% by weight of the total weight of the composition. Preferably, the amount of Marigold extract is 1% by weight of the total weight of the composition.

In another particular embodiment, in combination with any of the embodiments disclosed above, the lipogel of the invention for use as defined above comprises aloe vera in an amount comprised between 0.5 and 1% by weight of the total weight of the composition. Preferably, the amount of aloe vera is 1% by weight of the total weight of the composition.

In another particular embodiment, in combination with any of the embodiments disclosed above, the lipogel of the invention for use as defined above comprises centella asiatica extract in an amount comprised between 0.5 and 1.5 % by weight of the total weight of the composition. Preferably, the amount of aloe vera is 1% by weight of the total weight of the composition.

In a particular preferred embodiment, the lipogel of the invention for use as defined above comprises a) lidocaine hydrochloride in an amount of 2% by weight of the total weight of the composition; b) diclofenac sodium in an amount of 0.5 by weight of the total weight of the composition; c) the medium chain triglyceride in an amount comprised between 86 and 88% by weight of the total weight of the composition; d) the colloidal silica in an amount 6 % by weight of the total weight of the composition; e) the soy lecithin in an amount comprised between 1.5 and 3% by weight of the total weight of the composition; f) the Marigold extract in an amount of 1% by weight of the total weight of the composition; g) the aloe vera in an amount comprised between 0% and 1% by weight of the total weight of the composition; and h) the centella asiatica extract in an amount of 1% by weight of the total weight of the composition.

The pharmaceutical composition in the form of a topical lipogel and which comprises: a) a therapeutically effective amount of lidocaine or a pharmaceutically acceptable salt thereof; b) a therapeutically effective amount of diclofenac or a pharmaceutically acceptable salt thereof; c) a medium chain triglyceride; d) colloidal silica; e) soy lecithin; f) marigold extract; and g) aloe vera, as product *per se,* is also part of the invention.

The preferred embodiments for the first aspect of the invention are also preferred embodiments for this second aspect of the invention.

In particular, the lipogel may comprise lidocaine in the form of hydrochloride salt and diclofenac in the form of sodium salt. The pharmaceutical composition may also further comprise Centella asiatica extract.

Preferably, the amounts of each of the components of the lipogel of the present invention are the ones disclosed above, including any of the preferred and more preferred amounts.

Topical compositions of the present invention can be prepared according to methods well known in the state of the art. The appropriate excipients and/or carriers, and their amounts, can readily be determined by those skilled in the art according to the type of formulation being prepared.

The term "percentage (%) by weight" refers to the percentage of each ingredient of the combination or composition in relation to the total weight.

It is also part of the invention a kit for the treatment of pre- and post-operative pain in benign anorectal surgery comprising: a) a first pharmaceutical composition in the form of a lipogel comprising a therapeutically effective amount of lidocaine or a pharmaceutically acceptable salt thereof, and a therapeutically effective amount of diclofenac or a pharmaceutically acceptable salt thereof together with one or more pharmaceutically acceptable excipients or carriers; and b) a second pharmaceutical composition comprising lidocaine or a pharmaceutically acceptable salt thereof together with one or more pharmaceutically acceptable excipients or carriers. The kit includes instructions for the use of the first and the second pharmaceutical composition in the treatment of pre- and post-operative pain in benign anorectal surgery with the specific route of administration and the specific dosage regime disclosed above,

In a particular embodiment, the second pharmaceutical composition is in the form of lipogel and comprises the same pharmaceutically acceptable excipients than the ones used for the first pharmaceutical composition. All the preferred and particular embodiments of the first pharmaceutical composition with respect to the excipients and amounts are also preferred and particular embodiments for this composition.

The following examples and drawings are provided by way of illustration.

### BRIEF DESCRIPTION OF THE DRAWINGS

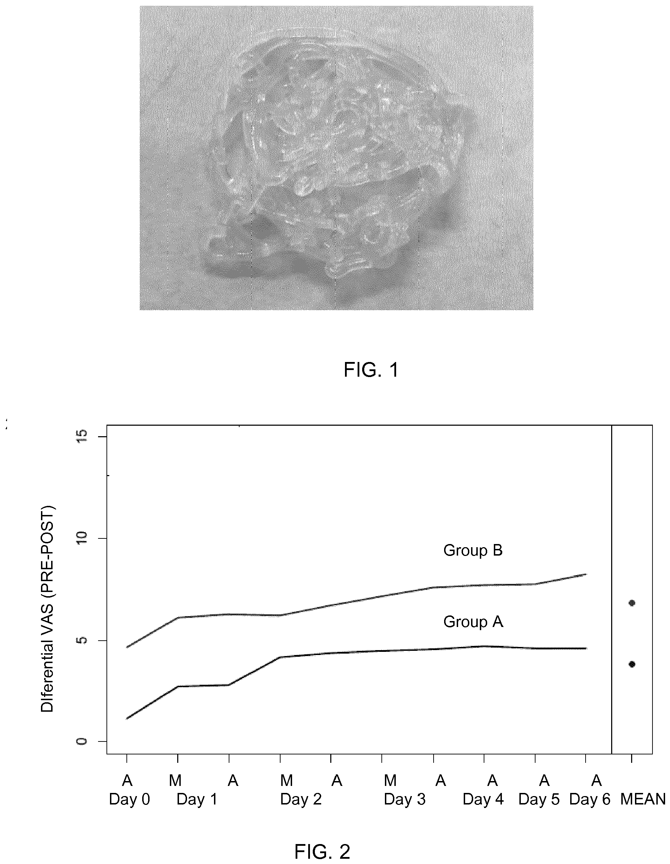

FIG. 1 shows the aspect of the lipogel of Example 4.
FIG. 2 shows VAS mean differential (pre-post gel administration) during the clinical trial (accumulated from day 0 to 7). Horizontal axis represent different measurement times and vertical axis the differential VAS values for the patients in groups A or B joined, accumulating the data from the day 0 until the day 6. Average of the means for each group is also shown, represented by a solid circle. A in the horizontal axis means afternoon, and M means morning.

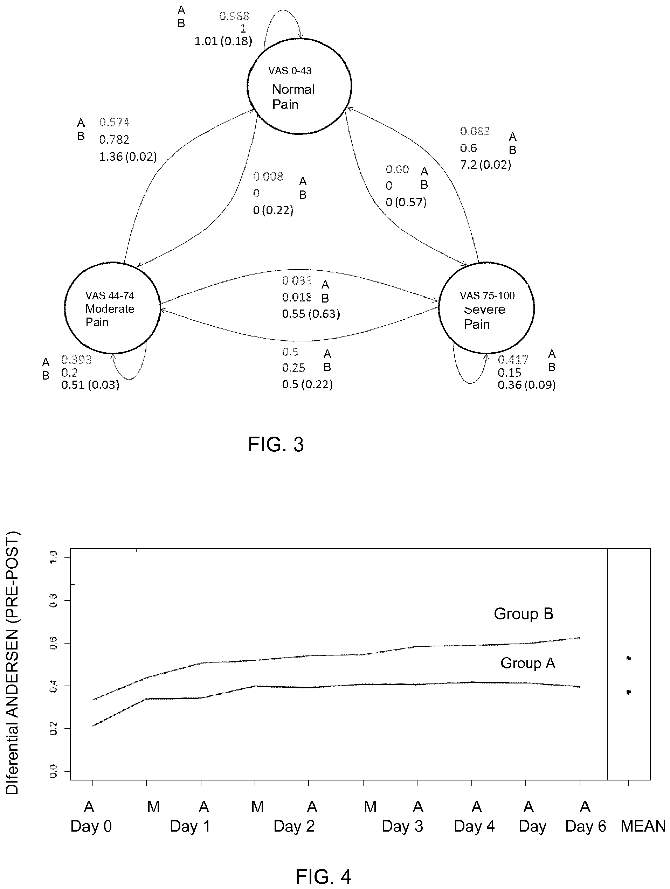

FiG. 3 shows the estimated probability of observing a change in the pain levels, considering three pain intervals (0-43, 44-74 and 75-100 in VAS scale) represented as nodes in a graph. For each node, three arrows arise to the three possible pain intervals, representing all possible situations. The probability of evolving from one pain interval to another is shown at each arrow: for treatment A and for treatment B, and the relative risk of B with respect to A (with a corresponding p-value).
FIG. 4 shows Andersen mean differential (pre-post gel administration) during the clinical trial (accumulated from day 0 to 7). Horizontal axis represent different measurement times and vertical axis the differential Andersen values for the patients in groups A or B, accumulating the data from the day 0 until the day 6. Average of the means for each group is also shown, represented by a solid circle. A in the horizontal axis means afternoon, and M means morning.

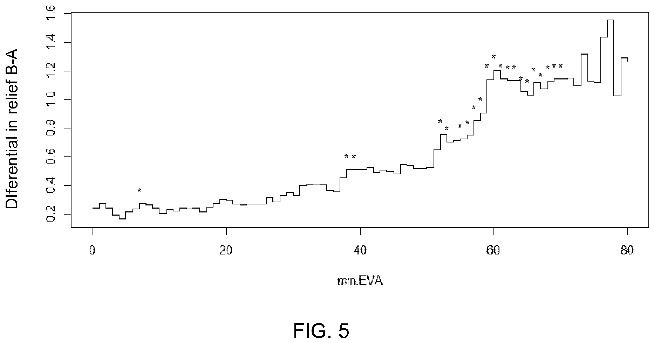

FIG.5 shows the analysis of the differential in the relief of pain estimated between groups A and B for different minimal thresholds in the VAS scale. The line shows the differential as estimated by a mixed linear model for each minimal VAS threshold, those significant are marked with an asterisk.

### EXAMPLES

**Components source**

| Compound | Supplier |
|---|---|
| Lidocaine hydrochloride | Laboratories Reig Jofre, S.A |
| Sodium diclofenac | Laboratories Reig Jofre, S.A |
| Marigold oil | Fagron |
| Centella asiatica extract | Fagron |
| Aloe vera | Fagron |
| Medium chain triglycerides | Fagron |
| Silica coloidal 200 | Fagron |
| Soy lecithin | Cargill |

Lipogel aspect: A visual inspection of color and general appearance, putting a quantity of sample on a glass plate.

Viscosity determination: A quantity of sample (a median droplet size) is set to scan in the center plate viscometer and proceeds to read. The viscosity is measured with a Broncofield Viscosimeter (Broncofield 2000 CAP), in the following conditions: Temperature 25°C spindle 0.5, , Speed 70 r.p.m., holt time 12 seconds and run time 12 seconds.

Extensibility determination: A quantity of the sample is set in the hole of the extensometer, which must be filled and flush. Then it is put the lid and on top of the lid a weight of 100 g is placed for 1 minute. After this time, the diameter of the circle is measured with a caliper. The result is the average of three measures. This value is used to calculate the Circle surface (S = π • r2).

Centrifugation: The purpose of this parameter is to force mechanical stress conditions of the formulation to test the stability and that does not present phase separation. Approximately 5 g of sample is weighed into a centrifuge tube. In another tube is added the same amount of water to compensate for the weight. They are centrifuged placing the centrifuge tubes symmetrically, at a speed of 5000 rpm for 15 minutes. If separation occurs phase, the speed is reduced to 1000 rpm.

Determination of water activity (aw):This assay is to determine the amount of free water of the composition with capacity of reacting. To determine the aw, a sufficient amount of sample is added to cover half of the capsule sample holder of the device and is placed in the lower chamber thereof; then it is proceeded to the reading.

pH Determination: 2 g of sample is weighed in a beaker and 18ml of deionized water are added (Concentration 10%), it is stirred magnetically for 10 minutes and then proceed to pH reading by triplicate.

### Examples 1-6: Formulations lidocaine hydrochloride + diclofenac sodium

**Formulations:**

| Example | 1 | 2 | 3 | 4 | 5 | 6 |
|---|---|---|---|---|---|---|
| Lidocaine hydrochloride | 2,0 g | 2,0 g | 2,0 g | 2,0 g | 2,0 g | 2,0 g |
| Diclofenac sodium | 0,5 g | 0,5 g | 0,5 g | 0,5 g | 0,5 g | 0,5 g |
| Silica colloidal 200 | 6,0 g | 6,0 g | 6,0 g | 6,0 g | 6,0 g | 6,0 g |
| Marigold extract | 1,0 g | 1,0 g | 1,0 g | 1,0 g | 1,0 g | 1,0 g |
| Centella asiatica extract | 1,0 g | 1,0 g | 1,0 g | 1,0 g | 1,0 g | - |
| Medium chain triglycerides | 86,5 g | 88,0 g | 85,5 g | 87.0 g | 86,0 g | 90.5g |
| Aloe vera | - | - | 1.0 g | 1.0 g | 1.0 g | 1,0 g |
| Soy lecithin | 3.0 g | 1.5 g | 3.0 g | 1.5 g | 2.5 g | 1.5 g |
| total | 100,0 g | 100,0 g | 100,0 g | 100,0 g | 100,0 g | 100,0g |

Preparation process: All components are weighed in the amounts indicated in the table above. The lidocaine hydrochloride was previously screened through a sieve of 90 microns mesh. Medium chain triglycerides were heated to 80 °C with a water bath. Colloidal silica was gradually added under magnetic stirring (750-1000 rpm) until a homogeneous dispersion is got. It was stirred with a glass rod when the consistency increased. It was cooled down to 30 °C while stirring. Subsequently, the following components were added and dispersed in the following order: lidocaine hydrochloride, diclofenac sodium, Centella asiatica extract, Marigold oil, and finally soy lecithin, and Aloe vera when appropriate. The mixture was stirred until a homogeneous mixture was obtained. The formulation is stored in tubes with an integrated cannula.

Appearance: The five formulations of Examples 1-6 have shown a good dispensability both of the active ingredients and of the excipients. The lipogel obtained is a yellow homogeneous gel, having an oleous and smooth texture when it is applied to the skin. The five formulations have a tixotropic flow since all show more consistency after 24 hours from their preparation and they show a more fluid texture when stirred.

### Example 7: Formulations lidocaine hydrochloride

| Example | 7 |
|---|---|
| Lidocaine hydrochloride | 2,0 g |
| Silica colloidal 200 | 6,0 g |
| Marigold extract | 1,0 g |
| Centella asiatica extract | 1,0 g |
| Medium chain triglycerides | 87,5g |
| Aloe vera | 1,0 g |
| Soy lecithin | 1.5 |
| Total | 100 |

Preparation process: It was followed the same process disclosed for examples 1-6 with the components of the previous table.

### Example 8: Characterization of the formulations of Examples 1-5 using the SeDeM diagram for lipogels in two conditions:

The SeDeM diagram for lipogels is a strategy of Quality by design (QbD) which search for robust products and processes. This methodology has been used to carry out the characterization (cf. J.M. Suñé et al., "New methodology of galenic preformulation to characterize substances in relation to its viability for compression: Se DeM Diagram", in Spanish, Clen. Technol. Pharm, 2005, vol. 15, pp. 125-136).
- Time cero: galenic controls are carried out to the lipogels 24 hours after its manufacture
- Stress assays: galenic controls are carried out to the lipogels after being submitted to temperature changes during a month as follows
   1° → 7 days at 30 °C
   2° → 2 days at 5 °C
   3° → 2 days at 22 °C
   4° → Centrifugal process: 1000 rpm during 15 minutes
   5° →2 days at 5 °C
   6° →3 days at 30 °C
   7° → 2 days at 22 °C

The characterization of both active ingredients, lidocaine hydrochloride and diclofenac sodium was considered satisfactory and appropriate to Ph. Eur. The excipients have resulted also viable in the pharmaceutical form.

| **Organoleptic features** | 10 | 10 | |
|---|---|---|---|
| Homogeneity | 2 | 2 | 2 |
| Coloration | 2 | 2 | 2 |
| Texture (over glass) | 2 | 2 | 2 |
| Absence of foam | 2 | 2 | 2 |
| Output of tube or cannula | 2 | 2 | 2 |

| **Viscosity** (viscosimeter Broonckfield 2000 CAP, at 25°C) | | | |
|---|---|---|---|
| Viscosity T₀ | 1.588,0 | 1.134,66 | 718 |
| Vicosity Tₑₜ | 1.595,3 | | |

| **Extensiveness (ε)** (extensometer Suñé Arbussà /del Pozo Ojeda) [mean surface in mm²] | | | |
|---|---|---|---|
| Extensiveness T₀ [surface (mm²)] | 502,0 | | 642,64 |
| Extensiveness Tₑₜ [surface (mm²)] | 516,3 | | |

| **Water activity** (aw) | | | |
|---|---|---|---|
| | 59 | 70 | 53 |

| **Centrifugation** | | | |
|---|---|---|---|
| Condition: 5000 rpm 15 min | Slight separation, oily | Slight separation, oily | Slight separation, oily |
| Condition: 1000 rpm 15 min | No phase separation | No phase separation | No phase separation |

The pH of formulation 4 is 7,023, which is near to the rectal pH (7.8-8).

### Example 9: Clinical assay to evaluate the efficacy and security of a hydro soluble topical rectal gel of lidocaine hydrochloride plus diclofenac sodium

Multicenter randomized double-blind trial to evaluate the analgesic efficacy and security of a hydro soluble topical rectal gel of CLIFE2 (lidocaine, referred as treatment A) respect CLIFE1 (lidocaine plus diclofenac, referred as treatment B) in the benign anorectal surgery.

The group of patients was formed by patients over 18 with benign anorectal surgery scheduled under subaracnoidea anesthesia with lidocaine that give their informed approval and do not present any intolerance or allergy to lidocaine or diclofenac.

The aim of the clinical trial was to compare the safety and efficacy of 300 mg lidocaine plus 75 mg diclofenac sodium and of 300 mg of lidocaine rectally administered, and demonstrate the superiority of the former in the decreased postoperative pain in benign anorectal surgery and the increase in the patient's satisfaction. The administration regime was twice per day during three days and once per day from the fourth day of treatment. The administered dose (two doses) is 15 gr of gel per day the first three postoperative days, and one dose from the fourth day, in both treatments during 7 consecutive days.

120 patients were randomly assigned to two groups (60 in each group). The rate of patients who did not complete the study was relatively small (6 in each group), showing no statistically significant differences between groups (p=1). The analysis presented here were generally obtained with the intention-to-treat population (ITT, 120 patients, 60 per group) even though for some analysis the results were obtained with the per-protocol population (ITT, 108 patients, 54 per group).

The results show that the treatment B (CLIFE1) shows a higher level of efficacy than treatment A (CLIFE2), reaching this difference the statistical significance despite the control treatment is also active. In addition, both treatments showed similar levels of security.

### Missing values evaluation

No statistically significant differences were observed in the percent of missing data between groups A and B (1.73% and 1.79% respectively, p=1), nor in the percentage of patients showing at least one missing value (15% y 10% respectively, p=0.58), nor in the mean number of pain evaluations in the trial (26.53±1.55 and 26.52±2.40 respectively, p=0.43).

### Cohort description and homogeneity analysis between groups before surgery

A significantly higher percentage of males was observed in group B with respect to group A (70% and 50% respectively, p=0.04). No differences with regards to race or education level were observed. The mean age for the included patients was 47.55±12.39 years, mean weight was 74.73±17.3 kg, mean height 167.72±9.79 cm and mean BMI 26.46±5.3, with no significant differences between groups. The distribution of the physical state as measured using ASA categories was similar between both groups (ASA percentages I, II and III being 50%, 46.67% and 3% in group A and 43.33%, 56.67% and 0% in group B, p=0.24), as for the presence of toxic habits, being the mean number of cigarettes 3.82±6.75 and 4.45±6.75 for groups A and B respectively (p=0.44) and the mean of alcohol diary consumption in grams 1.75±12.9 and 2.72±12.9 in groups A and B respectively (p=0.14). Results from the analytical measurements, clinical variables and variables referring to medical history did not show any statistically significant difference between groups, with the exception of the urea levels (means 5.18±1.25 and 5.59±1.25 for groups A and B respectively, p=0.05) and platelet levels (means 254.45±50 and 233.62±50 for groups A and B respectively, p=0.02). As result of this analysis the variables sex, urea and platelet levels are considered as potentially confounders and therefore used for adjusting the p-values in the statistical analysis.

### Evaluation of the efficacy with the main outcome (VAS)

The mean reduction of the pain (computing the difference pre-post application of the gel), observed during the three first days after the intervention, assessed by means of a VAS scale (0 to 100) was of 4.38 units for group A and 7.47 units for group B, being the difference of these reductions statistically significant (p=0.008) and maintaining the significance when adjusting for possible confounding factors (p=0.01). The mean reduction calculated using the data of all the clinical trial (6 days from the surgical intervention) was also higher in the group B (means 8.08 and 4.26 respectively) achieving the statistical significance (difference -3.922; Cl 95% -6.622, -1.221; p=0.004) even when adjusting by possible confounding factors (p=0.004). The analysis of the area under de curve of the VAS differential (pre-post) also showed higher values for the group B with respect to group A, reaching the statistical significance (difference -17.527; Cl 95% - 33.206, -1.847; p=0.02 until the third day and difference -32.724; Cl 95% -57.043, -8.404; p=0.006 until sixth day).

Furthermore, when considering three stages of pain, with relation to VAS (0-44, 45-74 and 75-100), the probability of evolving to stage of less pain after the application of the gel was 1.43 times higher in the group B with respect to group A (RR=1.43; Cl 95% 0.98, 2.09; p-value=0.06; adjusted p-value=0.04). Symmetrically, the probability of evolving to a worse stage of pain was 7.97 time higher in the group A with respect to group B (RR=7.97; Cl 95% 1, 63.54; p-value=0.02; adjusted p-value=0.04).

Moreover, the analysis of the relative risk in the probability of evolving to a better pain stage after the application for the group B with respect to group A, for different reductions considered in the VAS scale, revealed a monotonous increasing effect. Indeed, the probability of pain improvement was 25% higher in group B for any reduction considered (p=0.000006); 39% higher in group B for reductions higher than 5 units in the VAS scale (p=0.00004); 86% higher in group B for reductions higher than 30 units in the VAS scale (p=0.02); and even 226% higher in group B for reductions higher than 50 units in the VAS scale (p=0.03), clearly supporting a dose-effect type relationship. Sensitivity analysis shows similar results when analyzing the data from the per-protocol population. FIG. 2 and FIG. 3 illustrate the results obtained.

### Evaluation of the efficacy with the main outcome (VAS) by type of surgical intervention

The obtained results support that for hemorrhoids intervention the mean pain reduction was higher than the one observed for all the interventions, being the reduction in the group A of 5.72 units in the VAS scale and 9.93 units for group B, achieving the difference between groups statistical significance (difference -4.116; Cl 95% -7.946, -0.286; p=0.04), even when adjusting by potentially confounding variables (p=0.04). However, for fissure and fistula interventions, even though important reductions are observed, these do not achieve statistical significance. The area under the curve analysis on the VAS pre-post differential showed higher values in the group B with respect to group A, with higher differences for hemorrhoids, followed by fistulas and fissures. For the hemorrhoids interventions the probability of evolving from a high pain stage (VAS 75-100) to a low pain stage (0-44) was 6.37 times higher in the group B with respect to the group B (p=0.03 and adjusted p = 0.02). The probability of evolving to a worse pain stage after the application of the gel was 8.35 times higher in the group B with respect to the group A, being the relative risk statistically significant (CI 95% 1.05, 66.38; p-value=0.02 and adjusted p-value=0.02). The analysis of the relative risk associated to an improvement of the pain by the minimum decrement in the VAS pre-post differential revealed that for hemorrhoids interventions the effect was significant independently of the reduction, while for the fistula interventions significance is only achieved for reductions higher than 20 units and for the fissure intervention for reductions lower than 2 units.

### Evaluation of the efficacy with the main outcome (VAS) in the hemorrhoids interventions by their complexity (one, two or three hemorrhoidal bundles)

Even that some empirical evidences were observed suggesting some effect attributing a higher efficacy of treatment B with relation to treatment A in the reduction of the pain in the interventions of three hemorrhoidal bundles, followed by those of two and one hemorrhoidal bundle, none of them achieved statistical significance.

### Evaluation of the efficacy with secondary outcomes: Andersen scale

The mean reduction of the pain assessed using the Andersen scale, observed during the three first days after the intervention, was of 0.40 units in the group A and 0.58 units in the group B, being the difference in the reductions statistically significant (difference -0.19; Cl 95% -0.358, -0.021; p=0.04). The mean reduction computed when considering all the measurements in the trial (from the intervention until 6 days after) was also higher in the group B than for group A (means 0.6 and 0.39 respectively) reaching statistical significance (difference 0.235; Cl 95% -0.398, -0.073; p=0.01). FIG. 4 illustrates the results obtained.

### Evaluation of the efficacy with secondary outcomes: perceived relief

The mean perceived relief observed by the patients was similar between groups, no statistically significant differences were found (means 2.33 and 3.57 respectively in the groups A and B, p=0.17, adjusted p=0.15). Nevertheless, when considering pain levels in the VAS scale higher than 50-70, higher levels of perceived relief were observed in the group B with relation to the group A, reaching this difference the statistical significance, and observing higher differentials for higher levels of pain in VAS scale. The following table and FIG. 5 illustrates the results obtained.

Table: Analysis of the differences between groups A and B in the perceived pain relief since the last application, measured in a scale from 0 to 4. The differences are calculated using the data for each individual in the six days analyzed.

| Minimum threshold VAS | VAS value≥threshold | p-value | Beta (Diferential B-A) | IC 95% | |
|---|---|---|---|---|---|
| 51 | 95 | 0.050 | 0.65 | 0 | 1.3 |
| 52 | 91 | 0.030 | 0.76 | 0.08 | 1.43 |
| 53 | 85 | 0.040 | 0.71 | 0.03 | 1.39 |
| 54 | 82 | 0.050 | 0.72 | 0.01 | 1.42 |
| 55 | 78 | 0.040 | 0.72 | 0.02 | 1.43 |
| 56 | 77 | 0.040 | 0.75 | 0.04 | 1.46 |
| 57 | 75 | 0.020 | 0.85 | 0.16 | 1.55 |
| 58 | 72 | 0.020 | 0.91 | 0.18 | 1.63 |
| 59 | 70 | 0.002 | 1.14 | 0.46 | 1.82 |
| 60 | 69 | 0.001 | 1.21 | 0.52 | 1.89 |
| 61 | 66 | 0.003 | 1.14 | 0.42 | 1.87 |
| 62 | 64 | 0.006 | 1.13 | 0.36 | 1.9 |
| 63 | 64 | 0.006 | 1.13 | 0.36 | 1.9 |
| 64 | 60 | 0.020 | 1.06 | 0.23 | 1.89 |
| 65 | 59 | 0.020 | 1.03 | 0.22 | 1.84 |
| 66 | 51 | 0.020 | 1.12 | 0.24 | 1.99 |
| 67 | 48 | 0.020 | 1.07 | 0.16 | 1.99 |
| 68 | 45 | 0.020 | 1.13 | 0.19 | 2.07 |
| 69 | 43 | 0.030 | 1.14 | 0.13 | 2.16 |
| 70 | 43 | 0.030 | 1.14 | 0.13 | 2.16 |
| 71 | 38 | 0.050 | 1.15 | 0 | 2.29 |

### Safety analysis

The results of the analysis of the analytical variables did not show any statistically significance difference between groups A and B, with relation to the presence of side effects or values of the analytical variables out of the normality range. No association was found with the number of side effects and other variables related to the treatment. Globally, both treatments were revealed as safe, showing similar levels of safety.

### Homogeneity analysis between groups after the surgical intervention

No statistically significant differences were found between groups in the variables related to the physical exploration after the surgical intervention. Results of the biochemical and hematological analytic variables measured after the surgical intervention did not show any significant difference, with the exception of the urea levels (means 5.18±1.41 and 5.61±1.41 for groups A and B respectively, p=0.05) and platelets levels (means 266.48±54.23 and 248.08±54.23 for groups A and B respectively, p=0.02), similarly to the observed results before the surgical intervention.

### Other complementary analysis

Time until the first urination. No statistically significant differences in the time until the first urination were observed between groups A and B. No significant correlation was neither found in between the time until the first urination and the level of pain before the surgical intervention, measured using the VAS scale.

Analysis of the correlation between VAS and Andersen scales. A high level of correlation was observed between the pain measured with VAS and that measured with Andersen scale, being this correlation statistically significant (r=0.7, p=<0.00001 for group A and r=0.701, p<0.00001 for group B).

### REFERENCES CITED IN THE APPLICATION

- US2005/0256187;
- Abstract of the Scientific Information database (SID) of MR. Rafiei et al., "Comparison of Post Operative Analgesia with preoperative topical lidocaine gel and lubricant gel in hemorroidectomy operation", Iranian Journal of Surgery 2008, vol. 15, pp.28-33;
- C. Placer et al. "A single dose of intrarectal diclofenac reduces urinary retention after surgery for hemorrhoids. Results of a randomized controlled trial" in Spanish, Cir Esp 2008, vol. 83, pp. 301-5;
- Liedtke et al., "Pharmacological Concept for Topical Synergistic Analgesia of Peripheral Neuromuscular Pain", Drug Res 2006, vol. 56, pp.108-114;
- P. Anand et al., "Topical capsaicin for pain management: therapeutic potential and mechanisms of action of the new high-concentration capsaicin 8% patch", British Journal of Anaesthesia 2011, vol.107, pp. 490-502;
- http://www.mayoclinic.org/drugs-supplements/diltiazem-oral-route/side-effects/drg-20071775;
- J. T: Wright el al., "Antihypertensive efficacy of night-time graded-release diltiazem versus morning amlodipine in Africans Americans" AJH 2004, vol. 17, pp. 734-742, in particular table 4;
- G.Sandrine et al., Drug-induced and toxic myopathies. Best practices & Research Clinical Rheumathology 2003, vol. 17, pp. 877-907;
- J. Listing et al., "The risk of infections associated with rheumatoid arthritis, with its comorbidity and treatment", Rheumatology, 2013, vol. 52, pp. 53-61;
- W. G. Dixon et al., "The influence of systemic glucocorticoid therapy upon the risk of non-serious infection in older patients with rheumatoid arthritis: a nested case-control study" Ann Rheum Dis 2011, vol. 70, pp. 956-960;
- M.S. Lionakis et al., "Glucocorticoids and invasive fungal infections", Lancet 2003, vol. 362: pp.1828-38;
- J.M. Suñé et al., "New methodology of galenic preformulation to characterize substances in relation to its viability for compression: Se DeM Diagram", in Spanish, Clen. Technol. Pharm, 2005, vol. 15, pp. 125-136;
- Mojgan Rahimi et al., "Comparison of topical anesthetic cream (EMLA) and diclofenac suppository for pain relief after hemorrhoidectomy: a randomized clinical trial", Surg Today 2012, vol. 42; pp.1201-1205;
- B. Irer et al.,in "Diclofenac suppository administration in conjunction with lidocaine gel during transrectal ultrasound-guided prostate biopsy: prospective, randomized, placebo-controlled stuydy" Adult Urology 2005, vol. 66, pp.799-802.

## Claims

1. A pharmaceutical composition in the form of a lipogel comprising a therapeutically effective amount of lidocaine or a pharmaceutically acceptable salt thereof, and a therapeutically effective amount of diclofenac or a pharmaceutically acceptable salt thereof, together with one or more pharmaceutically acceptable excipients or carriers, for use in the treatment of pre- and post-operative pain in benign anorectal surgery of hemorrhoids, anal fistulas, anal fissure, proctitis, and perianal eczema, wherein the treatment comprises the topical rectal administration of the composition twice per day during three days and once per day from the fourth day of treatment.

2. The pharmaceutical composition for use according to claim 1, wherein the lidocaine is in the form of hydrochloride salt and the diclofenac is in the form of sodium salt.

3. The pharmaceutical composition for use according to any of the claims 1-2, wherein the pharmaceutical composition is in the form of a topical gel having a viscosity equal to or less than 2000 miliPa.

4. The pharmaceutical composition for use according to any of the claims 1-3, wherein the composition comprises 300 mg of lidocaine hydrochloride and 75 mg of diclofenac sodium.

5. The pharmaceutical composition for use according to any of the claims 1-4, further comprising the topical administration of a second pharmaceutical composition comprising lidocaine or a pharmaceutically acceptable salt thereof, together with one or more pharmaceutically acceptable excipients or carriers, where the lipogel is administered once up to 30' before a bowel movement and once after the bowel movement,

6. The pharmaceutical composition for use according to any of the claims 1-5, which is devoid of corticoids.

7. The pharmaceutical composition for use according to any of the claims 1-6, which has a pH of 5.2-7.2.

8. The pharmaceutical composition for use according to any of the claims 1-7, which comprises a medium chain triglyceride; colloidal silica; soy lecithin; Marigold extract; and optionally, aloe vera, as excipients or carriers.

9. The pharmaceutical composition for use according to claim 8, wherein
a) the lidocaine or the pharmaceutically acceptable salt thereof is present in an amount comprised between 1 and 3% by weight of the total weight of the composition;
b) the diclofenac or the pharmaceutically acceptable salt thereof is present in an amount comprised between 0.5 and 2% by weight of the total weight of the composition;
c) the medium chain triglyceride; is present in an amount comprised between 70 and 90% by weight of the total weight of the composition;
d) the colloidal silica; is present in an amount comprised between 3 and 8 % by weight of the total weight of the composition;
e) the soy lecithin; is present in an amount comprised between 1 and 6% by weight of the total weight of the composition;
f) the Marigold extract is present in an amount comprised between 0.5 and 2% by weight of the total weight of the composition; and
g) the aloe vera is present in an amount comprised between 0.5 and 2% by weight of the total weight of the composition.

10. The pharmaceutical composition for use according to any of the claims 8-9, further comprising Asiatic centellum extract.

11. A pharmaceutical composition which is in the form of a topical lipogel and which comprises:
a) a therapeutically effective amount of lidocaine or a pharmaceutically acceptable salt thereof;
b) a therapeutically effective amount of diclofenac or a pharmaceutically acceptable salt thereof;
c) a medium chain triglyceride;
d) colloidal silica;
e) soy lecithin;
f) Marigold extract; and
g) optionally, aloe vera.

12. The pharmaceutical composition according to claim 11, wherein
a) the lidocaine or the pharmaceutically acceptable salt thereof is present in an amount comprised between 1 and 3% by weight of the total weight of the composition;
b) the diclofenac or the pharmaceutically acceptable salt thereof is present in an amount comprised between 0.5 and 2% by weight of the total weight of the composition;
c) the medium chain triglyceride; is present in an amount comprised between 70 and 90% by weight of the total weight of the composition;
d) the colloidal silica; is present in an amount comprised between 3 and 8 % by weight of the total weight of the composition;
e) the soy lecithin; is present in an amount comprised between 1 and 6% by weight of the total weight of the composition;
f) the Marigold extract is present in an amount comprised between 0.5 and 2% by weight of the total weight of the composition; and
g) the aloe vera is present and is in an amount comprised between 0.5 and 2% by weight of the total weight of the composition.

13. The pharmaceutical composition according to any of the claims 11-12, wherein the lidocaine is in the form of hydrochloride salt and the diclofenac is in the form of sodium salt.

14. The pharmaceutical composition, according to any of the claims 11-13, further comprising Asiatic centellum extract.

15. A kit for use in the treatment of pre- and post-operative pain in benign anorectal surgery of hemorrhoids, anal fistulas, anal fissure, proctitis, and perianal eczema comprising:
a) A first pharmaceutical composition in the form of a lipogel comprising a therapeutically effective amount of lidocaine or a pharmaceutically acceptable salt thereof, and a therapeutically effective amount of diclofenac or a pharmaceutically acceptable salt thereof together with one or more pharmaceutically acceptable excipients or carriers
b) a second pharmaceutical composition comprising lidocaine or a pharmaceutically acceptable salt thereof together with one or more pharmaceutically acceptable excipients or carriers.

## Patentansprüche

1. Eine pharmazeutische Zusammensetzung in Form eines Lipogels umfassend eine therapeutisch wirksame Menge von Lidocain oder ein pharmazeutisch akzeptables Salz davon, und eine therapeutisch wirksame Menge von Diclofenac oder ein pharmazeutisch akzeptables Salz davon, zusammen mit einem oder mehreren pharmazeutisch akzeptablen Hilfsstoffen oder Trägersubstanzen, zur Verwendung in der Behandlung von präoperativen und postoperativen Schmerzen bei einer gutartigen anorektalen Chirurgie von Hämorriden, analen Fisteln, Analfissur, Proktitis und perianalem Ekzem, wobei die Behandlung die topische rektale Verabreichung der Zusammensetzung zweimal täglich für die Dauer von drei Tagen und einmal täglich ab dem vierten Tag der Behandlung umfasst.

2. Die pharmazeutische Zusammensetzung zur Verwendung nach Anspruch 1, wobei das Lidocain in Form von Hydrochloridsalz und das Diclofenac in Form von Natriumsalz ist.

3. Die pharmazeutische Zusammensetzung zur Verwendung nach einem der Ansprüche 1-2, wobei die pharmazeutische Zusammensetzung in Form von einem topischen Gel ist, das eine Viskosität von 2.000 milliPa, oder weniger, hat.

4. Die pharmazeutische Zusammensetzung zur Verwendung nach einem der Ansprüche 1-3, wobei die Zusammensetzung 300 mg von Lidocainhydrochlorid und 75 mg von Diclofenac-Natrium umfasst.

5. Die pharmazeutische Zusammensetzung zur Verwendung nach einem der Ansprüche 1-4, weiterhin umfassend die topische Verabreichung von einer zweiten pharmazeutischen Zusammensetzung umfassend Lidocain oder ein pharmazeutisch akzeptables Salz davon, zusammen mit einem oder mehreren pharmazeutisch akzeptablen Hilfsstoffen oder Trägersubstanzen, wobei das Lipogel einmal bis 30' vor einem Stuhlgang und einmal nach dem Stuhlgang verabreicht wird.

6. Die pharmazeutische Zusammensetzung zur Verwendung nach einem der Ansprüche 1-5, welche frei von Corticoiden ist.

7. Die pharmazeutische Zusammensetzung zur Verwendung nach einem der Ansprüche 1-6, welche einen pH-Wert von 5,2-7,2 hat.

8. Die pharmazeutische Zusammensetzung zur Verwendung nach einem der Ansprüche 1-7, welche folgendes umfasst: ein mittelkettiges Triglycerid; kolloidales Siliciumdioxid (Kieselsol); Sojalecithin; Ringelblumenextrakt ; und, wahlweise, Aloe Vera, als Hilfsstoffe oder Trägersubstanzen.

9. Die pharmazeutische Zusammensetzung zur Verwendung nach Anspruch 8, wobei
a) das Lidocain oder das pharmazeutisch akzeptable Salz davon in einer Menge von zwischen 1 und 3 Gew.-% des gesamten Gewichtes der Zusammensetzung vorhanden ist;
b) das Diclofenac oder das pharmazeutisch akzeptable Salz davon in einer Menge von zwischen 0,5 und 2 Gew.-% des gesamten Gewichtes der Zusammensetzung vorhanden ist;
c) das mittelkettige Triglycerid in einer Menge von zwischen 70 und 90 Gew.-% des gesamten Gewichtes der Zusammensetzung vorhanden ist;
d) das kolloidale Siliciumdioxid in einer Menge von zwischen 3 und 8 Gew.-% des gesamten Gewichtes der Zusammensetzung vorhanden ist;
e) das Sojalecithin in einer Menge von zwischen 1 und 6 Gew.-% des gesamten Gewichtes der Zusammensetzung vorhanden ist;
f) der Ringelblumenextrakt in einer Menge von zwischen 0,5 und 2 Gew.-% des gesamten Gewichtes der Zusammensetzung vorhanden ist; und
g) die Aloe Vera in einer Menge von zwischen 0,5 und 2 Gew.-% des gesamten Gewichtes der Zusammensetzung vorhanden ist.

10. Die pharmazeutische Zusammensetzung zur Verwendung nach einem der Ansprüche 8-9, weiterhin umfassend einen Extrakt aus Asiatischem Wassernabel.

11. Eine pharmazeutische Zusammensetzung, welche in Form von einem topischen Lipogel ist und welche folgendes umfasst:
a) eine therapeutisch wirksame Menge von Lidocain oder einem pharmazeutisch akzeptablen Salz davon;
b) eine therapeutisch wirksame Menge von Diclofenac oder einem pharmazeutisch akzeptablen Salz davon;
c) ein mittelkettiges Triglycerid;
d) kolloidales Siliciumdioxid;
e) Sojalecithin;
f) Ringelblumenextrakt; und
g) wahlweise, Aloe Vera.

12. Die pharmazeutische Zusammensetzung nach Anspruch 11, wobei
a) das Lidocain oder das pharmazeutisch akzeptable Salz davon in einer Menge von zwischen 1 und 3 Gew.-% des gesamten Gewichtes der Zusammensetzung vorhanden ist;
b) das Diclofenac oder das pharmazeutisch akzeptable Salz davon in einer Menge von zwischen 0,5 und 2 Gew.-% des gesamten Gewichtes der Zusammensetzung vorhanden ist;
c) das mittelkettige Triglycerid in einer Menge von zwischen 70 und 90 Gew.-% des gesamten Gewichtes der Zusammensetzung vorhanden ist;
d) das kolloidale Siliciumdioxid in einer Menge von zwischen 3 und 8 Gew.-% des gesamten Gewichtes der Zusammensetzung vorhanden ist;
e) das Sojalecithin in einer Menge von zwischen 1 und 6 Gew.-% des gesamten Gewichtes der Zusammensetzung vorhanden ist;
f) der Ringelblumenextrakt in einer Menge von zwischen 0,5 und 2 Gew.-% des gesamten Gewichtes der Zusammensetzung vorhanden ist; und
g) die Aloe Vera anwesend ist und in einer Menge von zwischen 0,5 und 2 Gew.-% des gesamten Gewichtes der Zusammensetzung ist.

13. Die pharmazeutische Zusammensetzung nach einem der Ansprüche 11-12, wobei das Lidocain in Form von Hydrochloridsalz und das Diclofenac in Form von Natriumsalz ist.

14. Die pharmazeutische Zusammensetzung nach einem der Ansprüche 11-13, weiterhin umfassend einen Extrakt aus Asiatischem Wassernabel.

15. Ein Kit zur Verwendung in der Behandlung von präoperativen und postoperativen Schmerzen bei einer gutartigen anorektalen Chirurgie von Hämorriden, analen Fisteln, Analfissur, Proktitis und perianalem Ekzem, umfassend:
a) eine erste pharmazeutische Zusammensetzung in Form von einem Lipogel umfassend eine therapeutisch wirksame Menge von Lidocain oder einem pharmazeutisch akzeptablen Salz davon, und eine therapeutisch wirksame Menge von Diclofenac oder einem pharmazeutisch akzeptablen Salz davon zusammen mit einem oder mehreren pharmazeutisch akzeptablen Hilfsstoffen oder Trägersubstanzen
b) eine zweite pharmazeutische Zusammensetzung umfassend Lidocain oder ein pharmazeutisch akzeptables Salz davon zusammen mit einem oder mehreren pharmazeutisch akzeptablen Hilfsstoffen oder Trägersubstanzen.

## Revendications

1. Une composition pharmaceutique en forme d'un lipogel comprenant une quantité thérapeutiquement efficace de lidocaine ou d'un sel pharmaceutiquement acceptable de celle-ci, et une quantité thérapeutiquement efficace de diclofénac ou un sel pharmaceutiquement acceptable de celui-ci, conjointement avec un ou plusieurs excipients ou véhicules pharmaceutiquement acceptables, pour l'utilisation dans le traitement de la douleur pré-opératoire et post-opératoire dans la chirurgie ano-rectale bénigne des hémorroïdes, des fistules anales, d'une fissure anale, de la proctite, de l'eczéma péri-anal, où le traitement comprend l'administration rectale topique de la composition deux fois par jour pendant trois jours et une fois par jour à partir du quatrième jour du traitement.

2. La composition pharmaceutique pour l'utilisation selon la revendication 1, dans laquelle la lidocaine est en forme de sel chlorhydrate et le diclofénac est en forme de sel de sodium.

3. La composition pharmaceutique pour l'utilisation selon l'une quelconque des revendications 1-2, dans laquelle la composition pharmaceutique est en forme d'un gel topique ayant une viscosité égale ou inférieure à 2.000 milliPa.

4. La composition pharmaceutique pour l'utilisation selon l'une quelconque des revendications 1-3, dans laquelle la composition comprend 300 mg de chlorhydrate de lidocaine et 75 mg de diclofénac sodium.

5. La composition pharmaceutique pour l'utilisation selon l'une quelconque des revendications 1-4, comprenant en outre l'administration topique d'une seconde composition pharmaceutique comprenant de la lidocaine ou un sel pharmaceutiquement acceptable de celle-ci, conjointement avec un ou plusieurs excipients ou véhicules pharmaceutiquement acceptables, où le lipogel est administré une fois jusqu'à 30' avant d'avoir des selles et une fois après avoir les selles.

6. La composition pharmaceutique pour l'utilisation selon l'une quelconque des revendications 1-5, qui est dépourvue de corticoïdes.

7. La composition pharmaceutique pour l'utilisation selon l'une quelconque des revendications 1-6, qui a un pH de 5,2-7,2.

8. La composition pharmaceutique pour l'utilisation selon l'une quelconque des revendications 1-7, qui comprend un triglycéride à chaîne moyenne ; de la silice colloïdale ; de la lécithine de soya ; un extrait de souci officinal ; et, facultativement, de l'aloe vera, comme des excipients ou des véhicules.

9. La composition pharmaceutique pour l'utilisation selon la revendication 8, dans laquelle
a) la lidocaine ou le sel pharmaceutiquement acceptable de celle-ci est présent(e) dans une quantité comprise entre 1 et 3 % en poids par rapport au poids total de la composition ;
b) le diclofénac ou le sel pharmaceutiquement acceptable de celui-ci est présent(e) dans une quantité comprise entre 0,5 et 2 % en poids par rapport au poids total de la composition ;
c) le triglycéride à chaîne moyenne est présent dans une quantité comprise entre 70 et 90 % en poids par rapport au poids total de la composition ;
d) la silice colloïdale est présente dans une quantité comprise entre 3 et 8 % en poids par rapport au poids total de la composition ;
e) la lécithine de soya est présente dans une quantité comprise entre 1 et 6 % en poids par rapport au poids total de la composition ;
f) l'extrait de souci officinal est présent dans une quantité comprise entre 0,5 et 2 % en poids par rapport au poids total de la composition ; et
g) l'aloe vera est présent dans une quantité comprise entre 0,5 et 2 % en poids par rapport au poids total de la composition.

10. La composition pharmaceutique pour l'utilisation selon l'une quelconque des revendications 8-9, comprenant en outre un extrait de *Centella asiatica.*

11. Une composition pharmaceutique qui est en forme d'un lipogel topique et qui comprend :
a) une quantité thérapeutiquement efficace de lidocaine ou un sel pharmaceutiquement acceptable de celle-ci ;
b) une quantité thérapeutiquement efficace de diclofénac ou un sel pharmaceutiquement acceptable de celui-ci ;
c) un triglycéride à chaîne moyenne ;
d) de la silice colloïdale ;
e) de la lécithine de soya ;
f) un extrait de souci officinal ; et
g) facultativement, de l'aloe vera.

12. La composition pharmaceutique selon la revendication 11, dans laquelle
a) la lidocaine ou le sel pharmaceutiquement acceptable de celle-ci est présent(e) dans une quantité comprise entre 1 et 3 % en poids par rapport au poids total de la composition ;
b) le diclofénac ou le sel pharmaceutiquement acceptable de celui-ci est présent dans une quantité comprise entre 0,5 et 2 % en poids par rapport au poids total de la composition ;
c) le triglycéride à chaîne moyenne est présent dans une quantité comprise entre 70 et 90 % en poids par rapport au poids total de la composition ;
d) la silice colloïdale est présente dans une quantité comprise entre 3 et 8 % en poids par rapport au poids total de la composition ;
e) la lécithine de soya est présente dans une quantité comprise entre 1 et 6 % en poids par rapport au poids total de la composition ;
f) l'extrait de souci officinal est présent dans une quantité comprise entre 0,5 et 2 % en poids par rapport au poids total de la composition ; et
g) l'aloe vera est présent et il est dans une quantité comprise entre 0,5 et 2 % en poids par rapport au poids total de la composition.

13. La composition pharmaceutique selon l'une quelconque des revendications 11-12, dans laquelle la lidocaine est en forme de sel chlorhydrate et le diclofénac est en forme de sel de sodium.

14. La composition pharmaceutique, selon l'une quelconque des revendications 11-13, comprenant en outre un extrait de *Centella asiatica.*

15. Un kit pour l'utilisation dans le traitement de la douleur pré-opératoire et post-opératoire dans la chirurgie ano-rectale bénigne des hémorroïdes, des fistules anales, d'une fissure anale, de la proctite, et de l'eczéma péri-anal comprenant :
a) une première composition pharmaceutique en forme d'un lipogel comprenant une quantité thérapeutiquement efficace de lidocaine ou un sel pharmaceutiquement acceptable de celle-ci, et une quantité thérapeutiquement efficace de diclofénac ou un sel pharmaceutiquement acceptable de celui-ci conjointement avec un ou plusieurs excipients ou véhicules pharmaceutiquement acceptables
b) une seconde composition pharmaceutique comprenant de la lidocaine ou un sel pharmaceutiquement acceptable de celle-ci conjointement avec un ou plusieurs excipients ou véhicules pharmaceutiquement acceptables.
